# EUROPÄISCHE PATENTSCHRIFT

(11) **EP 1 280 505 B1**
(45) Veröffentlichungstag und Bekanntmachung des Hinweises auf die Patenterteilung: **02.05.2007**
(21) Anmeldenummer: 01923464.0
(22) Anmeldetag: 03.05.2001
(51) Int. Cl.: A61Q 17/04, A61K 8/35, A61K 8/49, A61K 8/37

(54) **PHOTOSTABILISIERUNG VON DIBENZOYLMETHAN-DERIVATEN**
PHOTOSTABILIZATION OF DIBENZOYLMETHANE DERIVATIVES
PHOTOSTABILISATION DE DERIVES DE DIBENZOYLE-METHANE

(30) Priorität: 12.05.2000 CH 933002000
(43) Veröffentlichungstag der Anmeldung: 05.02.2003
(73) Patentinhaber: Ciba Specialty Chemicals Holding Inc., 4057 Basel (CH)
(72) Erfinder: BIRRENBACH, Gerd, CH-4616 Kappel (CH)
(86) Internationale Anmeldenummer: PCT/CH2001/000275
(87) Internationale Veröffentlichungsnummer: WO 2001/085123

(56) Entgegenhaltungen:
- EP-A- 0 845 261
- EP-A- 0 880 962
- DE-A- 19 756 921
- DE-A- 19 846 772

## Beschreibung

Die Erfindung betrifft die Verwendung von 2,4-Bis-[[4-(2-ethylhexyloxy)-2-hydroxy]phenyl]-6-(4-methoxyphenyl)-1,3,5-triazin zur Verbesserung der Photostabilität von Dibenzoylmethan-Derivaten sowie entsprechende kosmetische Zusammensetzungen zum Schutz gegen UV-Strahlung und die Herstellung solcher Zusammensetzungen.

Es besteht ein wachsender Bedarf an guten topischen Sonnenschutzmitteln, um die schädigenden Wirkungen von UV-Licht auf die menschliche Haut, wie Sonnenbrand, photoallergische Reaktionen, frühzeitige Hautalterung und Hautkrebs, zu verhindern. Die meisten Sonnenschutzmittel bieten heute einen guten Schutz gegen ultraviolette Strahlung im Wellenlängenbereich von 290-320 nm (UV-B-Bereich). Da zahlreiche, in den letzten Jahren durchgeführte Studien gezeigt haben, dass ultraviolette Strahlung im Wellenlängenbereich von 320-400 nm (UV-A-Bereich) einen signifikanten Beitrag zu den sonnenlicht-induzierten Schädigungen der Haut liefert, wird zunehmend auch ein angemessener Schutz gegen UVA-Strahlung verlangt. Zudem hat die Verfügbarkeit von Sonnenschutzmitteln mit hohem Sonnenschutzfaktor (sun protection factor, nachfolgend auch als SPF bezeichnet) insofern zu Bedenken geführt, als sich die Anwender länger in der Sonne aufhalten können und damit in vermehrtem Masse der UVA-Strahlung ausgesetzt sind.

Idealerweise sollte ein UV-Filter absorbierte UV-Strahlung rasch und effizient in harmlose Wärmeenergie umwandeln, ohne dass dabei der UV-Filter und dessen Schutzwirkung abgebaut oder die Funktion und Sicherheit des Sonnenschutzmittels beeinträchtigt wird. Während eine grosse Auswahl an geeigneten UVB-Filtern besteht, sind gute UVA-Absorber jedoch selten, da diese zumeist wenig wirksam oder ungenügend photostabil sind. Insbesondere werden Dibenzoylmethan-Derivate wie 4-tert-Butyl-4'-methoxydibenzoylmethan, der gebräuchlichste und im Handel beispielsweise unter der Bezeichnung Parsol 1789 (Givaudan, Schweiz) erhältliche UVA-Filter, unter der Einwirkung von Sonnenlicht relativ rasch abgebaut und verlieren damit ihre Schutzwirkung (R.M. Sayre et al., Allured's Cosmetics & Toiletries, 114 (5): 85-91, 1999). Zudem ist bekannt, dass die sonst photostabilen, als UVB-Filter wirksamen Zimtsäureester wie der häufig in Kombination mit Dibenzoylmethanen verwendete 4-Methoxyzimtsäure-2-ethylhexylester (meist als Octyl-methoxycinnamat bezeichnet), der beispielsweise unter dem Handelnamen Parsol MCX (LaRoche, Schweiz) erhältlich ist, nachfolgend auf die Photoreaktion des Dibenzoylmethan-Derivates mit diesem Cycloadditionsprodukte bilden können und damit destabilisiert werden.

In EP-A-0 815 834 wurden zur Verbesserung der Photostabilität von Dibenzoylmethan-Derivaten gewisse Amide, wie insbesondere N,N-Diethyl-methylbenzamide und N-Butyl-N-acetyl-3-aminopropionsäure-ethylester, vorgeschlagen. In EP-A-0 970 961 wird für den gleichen Zweck die Zugabe gewisser Siliziumverbindungen, die eine Benzylidencampher-Funktion aufweisen, offenbart. Ferner ist ein erster photostabiler UVA-Filter bekannt geworden, nämlich die unter der Bezeichnung Mexoryl SX (Chimex, Frankreich) erhältliche Terephthalylidendicamphersulfonsäure, die wasserlöslich ist und ein starkes Absorptionsmaximum bei 345 nm aufweist.

In DE-A-197 56 921 wurde ferner die Verwendung von synthetischem Bienenwachs zur Erhöhung der UV-A-Schutzleistung kosmetischer oder dermatologischer Zubereitungen vorgeschlagen, die mindestens eine übliche UV-A-Filtersubstanz und/oder eine Breitbandfiltersubstanz enthalten, wobei unter anderem auch ein O/W-Lotion ohne Daten erwähnt wird, die neben 12 weiteren Komponenten 2,4-Bis-[[4-(2-ethylhexyloxy)-2-hydroxy]phenyl]-6-(4-methoxyphenyl)-1,3,5-triazin und 4-tert-Butyl-4'-methoxydibenzoylmethan enthält.

In der EP 0 845 261 werden Sonnenschutzmittelzusammensetzungen offenbart, die mindestens a) ein Dibenzoylmethanderivat, b)ein Benzophenonderivat und c) ein Phenylaminotriazinderivat enthalten. Mit dieser Kombination lassen sich photostabile Sonnenschutzformulierungen herstellen.

Überraschenderweise wurde nun gefunden, dass der vor kürzem unter der Bezeichnung Tinosorb S (Ciba Speciality Chemicals, Schweiz) in den Handel gelangte Breitband-Filter 2,4-Bis-[[4-(2-ethylhexyloxy)-2-hydroxy]phenyl]-6-(4-methoxyphenyl)-1,3,5-triazin geeignet ist, die Photostabilität von Dibenzoylmethen-Derivaten wie 4-tert-Butyl-4'-methoxydibenzoylmethan erheblich zu verbessern, und dass damit auch eine Destabilisierung von Zimtsäureestern wie 4-Methoxyzimtsäure-2-athylhexylester weitestgehend vermieden werden kann. Zudem ergänzt 2,4-Bis-[[4-(2-ethylhexyloxy)-2-hydroxy]phenyl]-6-(4-methoxyphenyl)-1,3,5-triazin den Spektralbereich der Dibenzoylmethane und trägt damit zusätzlich zur Schutzwirkung von Produkten bei, die diese Verbindungen enthalten.

Die Erfindung betrifft daher die Verwendung von 2,4-Bis-[[4-(2-ethylhexyloxy)-2-hydroxy]phenyl]-6-(4-methoxyphenyl)-1,3,5-triazin zur Verbesserung der Photostabilität von Dibenzoylmethan-Derivaten. Insbesondere betrifft die Erfindung die Verwendung von 2,4-Bis-[[4-(2-ethylhexyloxy)-2-hydroxy]phenyl]-6-(4-methoxyphenyl)-1,3,5-triazin in oder zur Herstellung von einer kosmetischen Zusammensetzung, umfassend ein Dibenzoylmethan-Derivat, zwecks Verbesserung der Photostabilität des Dibenzoylmethan-Derivates. Die Stabilisierung kann grundsätzlich dadurch erreicht werden, dass man eine wirksame Menge an 2,4-Bis-[[4-(2-ethylhexyloxy)-2-hydroxy]phenyl]-6-(4-methoxyphenyl)-1,3,5-triazin mit dem Dibenzoylmethan-Derivat in Kontakt bringt bzw. in die Zusammensetzung einbringt.

Ferner betrifft die Erfindung kosmetische Zusammensetzungen (insbesondere sogenannte Sonnenschutzmittel oder Lichtschutzmittel) zur topischen Anwendung zum Schutz gegen UV-Strahlung, insbesondere Sonnenlicht, umfassend einen kosmetisch annehmbaren Träger, mindestens ein Dibenzoylmethan-Derivat, mindestens einen Zimtsäureester und eine wirksame Menge an 2,4-Bis-[[4-(2-ethylhexyloxy)-2-hydroxy]-phenyl]-6-(4-methoxyphenyl)-1,3,5-triazin. Die Herstellung dieser Zusammensetzungen kann erfindungsgemäss dadurch erfolgen, dass man das Dibenzoylmethan-Derivat, den Zimtsäureester und 2,4-Bis-[[4-(2-ethylhexyloxy)-2-hydroxy]phenyl]-6-(4-methoxyphenyl)-1,3,5-triazin in den kosmetisch annehmbaren Träger einbringt.

2,4-Bis-[[4-(2-ethylhexyloxy)-2-hydroxy]phenyl]-6-(4-methoxyphenyl)-1,3,5-triazin, das gemäss INCI-Nomenklatur als Bis Ethylhexyloxyphenol Methoxyphenyl Triazine bezeichnet oder im Rahmen der vorliegenden Erfindung auch als EHPT abgekürzt wird, ist - wie erwähnt - ein bekannter Breitband-Filter, der unter dem Namen Tinosorb S (Ciba Speciality Chemicals, Schweiz) im Handel erhältlich ist. Die erfindungsgemäss erhältlichen kosmetischen Zusammensetzungen können typischerweise etwa 0,1 bis 15 Gew.-%, vorzugsweise etwa 0,5 bis 10 Gew.-%, an 2,4-Bis-[[4-(2-ethylhexyloxy)-2-hydroxy]phenyl]-6-(4-methoxyphenyl)-1,3,5-triazin enthalten.

Als Dibenzoylmethan-Derivate eignen sich erfindungsgemäss grundsätzlich beliebige, als UV-Filter wirksame Dibenzoylmethan-Derivate, insbesondere alkyl- und/oder alkoxy-substituierte Dibenzoylmethane, wie 2-Methyldibenzoylmethan, 4-Methyldibenzoylmethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyldibenzoylmethan, 4,4'-Dimethoxydibenzoylmethan, 4-tert-Butyl-4'-methoxydibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan und 2,6-Dimethyl-4-tert-butyl-4'-methoxydibenzoylmethan. Bevorzugt verwendbare Dibenzoylmethan-Derivate sind das unter dem Namen Eusolex 8020 (Merck, Deutschland) erhältliche 4-Isopropyldibenzoylmethan und insbesondere das beispielsweise unter dem Namen Parsol 1789 (Givaudan, Schweiz) erhältliche 4-tert-Butyl-4'-methoxydibenzoylmethan. Typischerweise können die erfindungsgemäss erhältlichen kosmetischen Zusammensetzungen etwa 0,1 bis 10 Gew.-% an Dibenzoylmethan-Derivat enthalten, wobei Mengen von etwa 0,5 bis 5 Gew.-% in der Regel bevorzugt sind.

Da die Dibenzoylmethan-Derivate wie 4-tert-Butyl-4'-methoxydibenzoylmethan UVA-Filter mit geringer Absorption im UVB-Bereich sind, werden bei alleiniger Verwendung von Dibenzoylmethan-Derivaten nur geringe (und unter Sonneneinwirkung infolge Photoinstabilität zudem rasch abfallende) Sonnenschutzfaktoren und vergleichsweise hohe Werte des Verhältnisses der UVA-Extinktion (UVA absorbance) zur UVB-Extinktion (UVB absorbance; im Rahmen der vorliegenden Erfindung wird das Verhältnis auch als UVA/UVB-Verhältnis bezeichnet; vgl. R. Stokes in: B. Gabard et al., Dermatopharmacology of Topical Preparations, Springer-Verlag Berlin, 2000, Seiten 365-382) erhalten. Durch Verwendung der als UVB-Filter wirkenden Zimsäureester, wie 4-Methoxyzimtsäure-2-ethylhexylester und 4-Methoxyzimtsäure-isoamylester, in Kombination mit Dibenzoylmethan-Derivaten können zwar die Sonnenschutzfaktoren anfänglich stark erhöht und UVA/UVB-Verhältnisse um oder unter 1 erreicht werden. Infolge der Photoinstabilität der Dibenzoylmethan-Derivate und der durch diese bedingten Destabilisierung der Zimtsäureester geht jedoch die Schutzwirkung rasch verloren.

Durch Verwendung von Dibenzoylmethan-Derivaten in Kombination mit 2,4-Bis-[[4-(2-ethylhexyloxy)-2-hydroxy]-phenyl]-6-(4-methoxyphenyl)-1,3,5-triazin können hingegen lang anhaltende, hohe Sonnenschutzfaktoren und stabile UVA/UVB-Verhältnisse von beispielsweise etwa 0,6 bis 1,5, insbesondere etwa 0,8 bis 1,1, d.h. ein gleichmässiger und anhaltender Schutz sowohl im UV-A- als auch im UV-B-Bereich, erreicht werden.

Zudem wird eine Destabilisierung von Zimtsäureestern in Gegenwart von Dibenzoylmethan-Derivaten durch 2,4-Bis-[[4-(2-ethylhexyloxy)-2-hydroxy]phenyl]-6-(4-methoxyphenyl)-1,3,5-triazin weitgehend vermieden, womit erfindungsgemäss auch photostabile kosmetische Zusammensetzungen ermöglicht werden, die einen (als UVB-Filter wirksamen) Zimtsäureester in Kombination mit einem Dibenzoylmethan-Derivat enthalten. Gemäss einem bevorzugten Aspekt der vorliegenden Erfindung kann daher 2,4-Bis-[[4-(2-ethylhexyloxy)-2-hydroxy]phenyl]-6-(4-methoxyphenyl)-1,3,5-triazin in oder zur Herstellung einer kosmetischen Zusammensetzung, umfassend ein Dibenzoylmethan-Derivat und einen Zimtsäureester, verwendet werden bzw. die kosmetische Zusammensetzung einen kosmetisch annehmbaren Träger, mindestens ein Dibenzoylmethan-Derivat, mindestens einen Zimtsäureester, wie 4-Methoxyzimtsäure-2-ethylhexylester oder 4-Methoxyzimtsäure-isoamylester, und eine wirksame Menge an 2,4-Bis-[[4-(2-ethylhexyloxy)-2-hydroxy]phenyl]-6-(4-methoxyphenyl)-1,3,5-triazin umfassen. Der Gehalt an Zimtsäureester kann in den erfindungsgemäss erhältlichen kosmetischen Zusammensetzungen typischerweise etwa 0,1 bis 15 Gew.-%, vorzugsweise etwa 0,5 bis 10 Gew.-%, betragen.

Die erfindungsgemässe Kombination von 2,4-Bis-[(4-(2-ethylhexyloxy)-2-hydroxy]phenyl]-6-(4-methoxyphenyl)-1,3,5-triazin mit Dibenzoylmethan-Derivaten eignet sich grundsätzlich zur Verwendung in allen gebräuchlichen Anwendungsformen, insbesondere flüssige oder halbfeste Formen, die sich zur Auftragung auf die Haut und/oder im Haar eignen, wie wässrige, ölige oder alkoholische Lösungen, hydrophile (wässrige) oder lipophile (ölige) Gele, Emulsionen vom Typ O/W (Öl-in-Wasser-Emulsion), W/O (Wasser-in-Öl-Emulsion), W/O/W oder O/W/O sowie Cremen oder Lotionen, kolloidale Systeme wie Mikroemulsionen, Nanoemulsionen, gemischt micellare Lösungen, liposomale Systeme, Nanosuspensionen etc. und hydrophile oder lipophile Suspensionen. Die Herstellung kann in an sich bekannter Weise unter Verwendung üblicher kosmetischer Trägermaterialien erfolgen, wobei je nach Zusammensetzung und Konsistenz kosmetische Produkte in Form einer Creme, Lotion, Milch oder Paste, eines Balsams, Sprays, Gels, Stiftes etc. erhalten werden können; entsprechende Anhaltspunkte finden sich beispielsweise in Cosmetics & Toiletries 105: 91-94, 1990 und 113: 83-98, 1998 sowie in Lehrbüchern und Standardwerken der Kosmetik.

Die erfindungsgemäss erhältlichen kosmetischen Zusammensetzungen können vorzugsweise Emulsionen sein, insbesondere O/W-Emulsionen oder W/O-Emulsionen. Besonders bevorzugt sind Emulsionen, die Phospholipid enthalten, insbesondere O/W-Emulsionen mit Phospholipid, da sich gezeigt hat, dass dadurch die Photostabilität weiter verbessert und auch eine Verbesserung der Hautpenetration und der Wasserfestigkeit erreicht werden kann.

Bevorzugte Öl-in-Wasser-Emulsionen umfassen neben den UV-Filtern typischerweise (a) eine wässrige Phase, enthaltend Wasser und übliche Exzipientien wie Verdickungs- oder viskositätserhöhende Mittel (z.B. Carbomer), Feuchthalte- oder hydratisierende Mittel (z.B. Sorbitol und/oder Glycerin), Puffersubstanzen im pH-Bereich von etwa 4 bis 8, vorzugsweise etwa 5,5 bis 7,0, oder Mittel zur Einstellung des pH auf diesen Wert (z.B. Tromethamin oder Triethanolamin) und Konservierungsmittel (z.B. Parabene wie Methylparaben und/oder Propylparaben); (b) Emulgiermittel, typischerweise eine Mischung von oberflächenaktiven Mitteln, wie Polysorbat 20, Sorbitanlaurat, Cetearylalkohol und/oder Acrylat/(C₁₀₋₃₀-Alkyl)acrylat-Crosspolymere; und (c) eine lipophile Phase, umfassend pflanzliche, tierische oder synthetische Öle (z.B. Silicone) und/oder lipophile Lösungsmittel, wie Dicaprylylmaleat, Dimethicon, (C₁₂₋₁₅-Alkyl)benzoat und/oder Triglyceride von C₈₋₁₈-Fettsäuren, Ethylhexylpalmitat und Paraffin.

Bevorzugte Wasser-in-Öl-Emulsionen umfassen neben den UV-Filtern typischerweise (a) eine wässrige Phase, umfassend Wasser und übliche Exzipientien wie Natriumlactat und Milchsäure sowie Konservierungmittel (z.B. Parabene wie Methylparaben und/oder Propylparaben); (b) Emulgiermittel wie PEG-7-hydriertes Ricinusöl (Polyethylenglycolderivat von hydriertem Ricinusöl mit im Mittel 2 Mol Ethylenoxid), Methoxy-PEG-22/Dodecylglycol-Copolymer und/oder ethoxylierte Glycerinsorbitanester gesättigter Fettsäuren; und (c) eine lipophile Phase, umfassend pflanzliche, tierische oder synthetische Öle (z.B. Silicone) und/oder lipophile Lösungsmittel, wie Myristyllactat, Triglyceride von C₈₋₁₈-Fettsäuren, Paraffin und/oder Dimethicon.

Emulsionen mit Phospholipid können eine ähnliche Zusammensetzung aufweisen wie die beschriebenen O/W- oder W/O-Emulsionen, wobei aber mindestens ein Emulgator ein Phospholipid, vorzugsweise Lecithin ist. Die Zugabe von Phospholipid modifiziert die Eigenschaften der lipophilen Phase, wodurch liposomale Strukturen gebildet werden können und die Hautpenetration und die Wasserfestigkeit verbessert werden. Bevorzugte Öl-in-Wasser-Emulsionen mit Phospholipid umfassen neben den UV-Filtern typischerweise (a) eine wässrige Phase, enthaltend Wasser und übliche Exzipientien wie Verdickungs- oder viskositätserhöhende Mittel (z.B. Carbomer), Feuchthalte- oder hydratisierende Mittel (z.B. Sorbitol und/oder Glycerin), Puffersubstanzen im pH-Bereich von etwa 4 bis 8, vorzugsweise etwa 5,5 bis 7,0, oder Mittel zur Einstellung des pH auf diesen Wert (z.B. Tromethamin) und Konservierungsmittel (z.B. Parabene wie Methylparaben und/oder Propylparaben); (b) Emulgiermittel, insbesondere mindestens ein Phospholipid wie Lecithin, gegebenenfalls in Kombination mit weiteren Emulgatoren wie Stearinsäure und/oder Triceteareth-4-phosphat; und (c) eine lipophile Phase, umfassend pflanzliche, tierische oder synthetische Öle (z.B. Silicone) und/oder lipophile Lösungsmittel, wie Dicaprylylmaleat, Dimethicon, (C₁₂₋₁₅-Alkyl)benzoat und/oder Triglyceride von C₈₋₁₈-Fettsäuren, Ethylhexylpalmitat und Paraffin. Beispielsweise kann eine solche Formulierung Wasser, Ethanol, Carbomer, Triethanolamin oder Tromethamin 20, Aloe Vera Gel, Sorbitol, Cetylphosphat, Cetylalkohol, Dicaprylylmaleat, Lecithin, Dimethicon, Tocopherolacetat, Methylparaben, Propylparaben, 2,4-Bis-[[4-(2-ethylhexyloxy)-2-hydroxy]phenyl]-6-(4-methoxyphenyl)-1,3,5-triazin, 4-tert-Butyl-4'-methoxydibenzoylmethan, 4-Methoxyzimtsäure-2-ethylhexylester und gewünschtenfalls weitere UV-Filter enthalten.

Die Herstellung dieser und weiterer liposomaler Formulierungen mit Phospholipid kann in an sich bekannter Weise erfolgen, beispielsweise nach den in WO-A-89/11850 und US-A-5 269 979 beschriebenen Methoden. Es hat sich jedoch gezeigt, dass geeignete Formulierungen in der Regel auch auf einfachere Weise dadurch erhalten werden können, dass man bei der Herstellung der Emulsion eine Lösung des Phospholipids in einem Alkohol, vorzugsweise Ethanol, zur lipophilen Phase zumischt und dann in an sich bekannter Weise eine Emulsion mittels Homogenisierung mit der wässrigen Phase auf geeigneten Anlagen herstellt.

Die erfindungsgemäss erhältlichen kosmetischen Zusammensetzungen können ferner weitere UV-Filter enthalten, beispielsweise Benzophenone wie 2-Hydroxy-4-methoxybenzophenon (Oxybenzon) oder 2-Hydroxy-4-methoxybenzophenon-5-sulfonsäure (Sulisobenzon); p-Aminobenzoesäure oder Derivate wie 4-Bis(2-hydroxypropyl)aminobenzoesäure-ethylester, Octyldimethyl-p-aminobenzoat, 4-Dimethylaminobenzoesäure-2-ethylhexylester, ethoxyliertes Ethyl-4-aminobenzoat oder 4-Bis(polyethoxy)aminobenzoesäure-poly-oxyethylester, Campherderivate wie 3-(4'-Methylbenzyliden)campher, 3-Benzylidencampher, Benzylidencamphersulfonsäure oder Terephthalylidendicamphersulfonsäure, Benzimidazol-Derivate wie die wasserlösliche 2-Phenylbenzimidazol-5-sulfonsäure; Salicylate wie 3,3,5-Trimethylcyclohexylsalicylat (Homosalatum), Salicylsäure-2-ethylhexylester (Octylsalicylat) oder 4-Isopropylbenzylsalicylat oder 2,4,6-Trianilino(p-carbo-2'-ethylhexyl-1'-oxy)-1,3,5-triazin; 3-(4'-Trimethylammonium)-benzyliden-bornan-2-on-methylsulfat oder 2-Cyan-3,3-diphenyl-acrylsäure-(2-ethylhexylester). (Octocrylene).

Zusätzlich zu den UV-Filtern können die erfindungsgemäss erhältlichen kosmetischen Zusammensetzungen gewünschtenfalls auch Mikropigmente oder Nanopigmente (Pigmente in mikronisierter Form) als sogenannte Sonnenblocker enthalten. Geeeignete Materialien, Teilchengrössen und Methoden sind beispielsweise in EP-A-0 433 086 und durch M.W. Anderson et al. in N.J. Lowe et al., Sunscreens: Development, Evaluation, and Regulatory Aspects, Marcel Dekker Inc., New York, 1997, Seiten 353-397 beschrieben. Vorzugsweise können als Mikropigmente Titandioxid mit einer Teilchengrösse < 35 µm, insbesondere < 10 µm, und/oder Zinkoxid mit einer Teilchengrösse < 50 µm, insbesondere < 20 µm, verwendet werden.

Die erfindungsgemäss erhältlichen kosmetischen Zusammensetzungen können ferner weitere, in kosmetischen Präparaten gebräuchliche Exzipientien enthalten, wie beispielsweise Antioxidantien, Parfümöl, Hautpflegemittel, Vitamine, filmbildende Mittel und/oder die Wasserbeständigkeit verbessernde Mittel, z.B. Biotin, Dexpanthenol, Aloe Vera Gel, Tocopherolactat, Acrylat/ (C₁₀₋₃₀-Alkyl) acrylat-Crosspolymer, PVP-Eicosen-Copolymer und dergleichen.

Die vorliegende Erfindung wird durch die nachfolgenden Beispiele weiter veranschaulicht, in denen die für Komponenten verwendeten Bezeichnungen und Abkürzungen folgende Bedeutungen haben:
Carbomer ist ein mit einem Allylether von Pentaerythritol, einem Allylether von Sucrose oder einem Allylether von Propylen vernetztes Homopolymer von Acrylsäure, das beispielsweise unter der Bezeichnung Carbopol 940 (BF Goodrich, USA) im Handel erhältlich ist;
Tromethamin 20% bezeichnet eine wässrige Lösung, enthaltend 20 Gew.-% 2-Amino-2-(hydroxymethyl)-1,3-propandiol;
Sorbitol 70% bezeichnet eine wässrige Lösung, enthaltend 70 Gew.-% Sorbitol;
Natriumlactat-Lösung ist eine wässrige Lösung, enthaltend 50 Gew.-% Natriumlactat;
Polysorbat 20 ist ein Gemisch von Laurinsäureestern von Sorbitol und Sorbitolanhydriden, das vorwiegend aus dem mit etwa 20 Mol Ethylenoxid kondensierten Monoester besteht und auch als Polyoxyethylen(20)-sorbitanmonolaurat bekannt ist;
Sorbitanlaurat ist beispielsweise als Tween 20 (Uniqema Americas, USA) im Handel erhältlich;
Cetearylalkohol bezeichnet ein Gemisch von Fettalkoholen, das vorwiegend aus Cetylalkohol und Stearylalkohol besteht und beispielsweise unter den Namen Hyfatol CS (Aarhus, Dänemark) erhältlich ist;
Triceteareth-4-phosphat ist ein Gemisch von Mono-di-tri-(alkyltetraglycolether)-O-phosphorsäureestern, in denen die Alkylreste vorwiegend 16-18 C-Atome aufweisen, und das unter der Bezeichnung Hostaphat KW 340 N (Clariant GmbH, Deutschland) im Handel erhältlich ist;
PEG-7-hydr. Ricinusöl bezeichnet ein Polyethylenglycolderivat von hydriertem Ricinusöl mit im Mittel 7 Mol Ethylenoxid pro Mol Ricinusöl, das beispielsweise als Arlacel 989 (Uniqema Americas, USA) erhältlich ist;
Methoxy-PEG-22/Dodecylglycol-Copolymer ist ein Polymer der Formel CH₃O(CH₂CH₂)ₓ[CH₂CH(C₁₀H₂₁)O]_{y}H, worin x einen Mittelwert von 22 und y einen Mittelwert von 7 hat, und das beispielsweise unter der Bezeichnung Elfacos E 200 (Akzo Nobel, Niederlande) im Handel erhältlich ist;
Arlacel 582 (ICI, Grossbritannien) ist ein ethoxylierter Glycerinsorbitanester gesättigter Fettsäuren;
Dicaprylylmaleat bezeichnet den Diester von Caprylalkohol und Maleinsäure;
Lecithin ist beispielsweise unter der Bezeichnung Phospholipon (Nattermann, Deutschland) im Handel erhältlich;
Dimethicon ist ein Gemisch vollständig methylierter linearer Polysiloxan-Polymere mit Trimethylsiloxy-Endgruppen, das beispielsweise unter dem Namen AEC Dimethicone (350 CS) (A & E Connock, Grossbritannien) im Handel erhältlich ist;
(C_{12-C15}-Alkyl)benzoat bezeichnet ein Gemisch von Alkylbenzoaten mit 12-15 C-Atomen im Alkylrest, das beispielsweise unter dem Namen Finsolv TN (Finetex, USA) im Handel erhältlich ist;
als Triglycerid mittlerer Kettenlänge wurde ein vorwiegend aus Triglyceriden von C₈₋₁₂-Fettsäuren bestehendes Gemisch wie Miglyol 812 Neutralöl (Condea Chemie, Deutschland) verwendet;
Paraffin (Mineralöl) bezeichnet eine Mischung von Kohlenwasserstoffen entsprechend dem Begriff "Paraffinum Liquidum" gemäss der Europäischen Pharmakopöe;
Methylparaben bezeichnet den p-Hydroxybenzoesäure-methylester;
Propylparaben bezeichnet den p-Hydroxybenzoesäure-n-propylester;
AVB oder Avobenzon ist der UVA-Filter 4-t-Butyl-4'-methoxydibenzoylmethan und ist beispielsweise unter der Bezeichnung Parsol 1789 (LaRoche, Schweiz) erhältlich;
OMC oder Octyl-methoxycinnamat ist der UVB-Filter 4-Methoxyzimtsäure-2-ethylhexylester und ist beispielsweise unter der Bezeichnung Parsol MCX (LaRoche, Schweiz) erhältlich;
EHPT oder Bis Ethylhexyloxyphenol Methoxyphenyl Triazine ist der Breitbandfilter 2,4-Bis-[[4-(2-ethylhexyloxy)-2-hydroxy]phenyl]-6-(4-methoxyphenyl)-1,3,5-triazin und ist unter der Bezeichnung Tinosorb S (Ciba Speciality Chemicals, Schweiz) im Handel erhältlich.

Die in vitro-Bestimmung des Sonnenschutzfaktors erfolgte nach der Methode von Diffey und Robson (B.L. Diffey und J. Robson, J. Soc. Cosmet. Chem. 40: 127-133, 1989; B.L. Diffey et al., Eur. J. Dermatol. 7: 226-228, 1997) mittels eines SPF-Messsystems mit 150 W-Xenon-Lampe und 35 x 35 mm-Quarzplatten der Firma Glen Spectra (Grossbritannien) und unter Verwendung von Transpore-Bändern (Minnesota Mining & Manufacturing, USA). Das zu untersuchende Sonnenschutzprodukt wurde jeweils in einer Menge von 2 mg/cm² auf zwei Quarzplatten aufgetragen. Eine Platte wurde mit UV-Licht der Xenon-Lampe bestrahlt, das optisch filtriert wurde, um Sommer-Sonnenlicht zu simulieren (Multiport Solar UV-Simulator Model 501, Solar Light, Philadelphia, USA). Die andere Platte wurde als Kontrolle unbestrahlt gelassen (Aufbewahrung im Brutschrank bei 30°C). Die UV-Strahlungsintensität wurde auf 5,5 mW/cm² eingestellt. Nach Bestrahlung mit 30 MED (1 MED = 25 mJ/cm² = minimum erythemal dose) wurde die spektrale Transmission der bestrahlten Probe und unbestrahlten Kontrollprobe gemessen.

Ergänzend wurde das Verhältnis der UVA-Extinktion zur UVB-Extinktion (im Rahmen der vorliegenden Erfindung auch als UVA/UVB-Verhältnis bezeichnet) nach der von R. Stokes (in: B. Gabard et al., Dermatopharmacology of Topical Preparations, Springer-Verlag Berlin, 2000, Seiten 365-382) beschrienen Methode bestimmt, und ferner wurden die Sonnenschutzprodukte nach Solubilisierung von der Quarzplatte mittels Hochdruck-Flüssigkeitschromatographie untersucht.

### Beispiel 1

### (O/W-Emulsionen)

Zur Herstellung der in Tabelle 1 zusammengefassten O/W-Emulsionen wurde jeweils Carbomer in Wasser dispergiert, die Dispersion mit Sorbitol 70% und Methylparaben versetzt und die erhaltene wässrige Phase auf 80°C erwärmt. Polysorbat 20, Sorbitanlaurat, Cetearylalkohol, Dicaprylylmaleat, Dimethicon, (C₁₂₋₁₅-Alkyl)benzoat, Propylparaben und die UV-Filter AVB, OMC und/oder EHPT wurden in einem separaten Kessel gemischt, auf 80°C erwärmt und dann zur erwärmten wässrigen Phase zugegeben. Das Gemisch wurde während etwa 2 Minuten homogenisiert, dann auf Umgebungstemperatur gekühlt, durch Zugabe von Tromethamin 20% auf einen pH-Wert von 6,0-6,5 eingestellt und nochmals etwa 2 Minuten homogenisiert.

Nach den oben beschriebenen in vitro-Methoden wurde der Einfluss von UV-Licht (30-minütige Bestrahlung mit 30 MED) auf die Schutzeigenschaften und die Stabilität der O/W-Emulsionen untersucht. Die gemessenen Sonnenschutzfaktoren und UVA/UVB-Verhältnisse sowie deren prozentuale Änderungen (bezogen auf den Anfangswert) und der mittels HPLC bestimmte prozentuale Abbau von AVB und/oder OMC sind in Tabelle 2 angegeben. Die angegebenen Werte sind jeweils Mittelwerte (inklusive Streuungen) aus 3 Messungen.

**Tabelle 1**

| O/W-Formulierungen: Zusammensetzungen in Gew.-% | | | | | | | | | | | | | |
|---|---|---|---|---|---|---|---|---|---|---|---|---|---|
| Komponenten | 1 A | 1 B | 1 C | 1 D | 1 E | 1 F | 1 G | 1 H | 1 I | 1 J | 1 K | 1 L | 1 M |
| Wasser | 74,7% | 72,2% | 67,2% | 72,1% | 67,1% | 62,2% | 69,7% | 62,2% | 57,2% | 79,6% | 77,2% | 74,7% | 67,2% |
| Carbomer | 0,2% | 0,2% | 0,2% | 0,2% | 0,2% | 0,2% | 0,2% | 0,2% | 0,2% | 0,2% | 0,2% | 0,2% | 0,2% |
| Tromethamin 20% | 0,7% | 0,7% | 0,7% | 0,8% | 0,8% | 0,7% | 0,7% | 0,7% | 0,7% | 0,8% | 0,7% | 0,7% | 0,7% |
| Sorbitol 70% | 3,0% | 3,0% | 3,0% | 3,0% | 3,0% | 3,0% | 3,0% | 3,0% | 3,0% | 3,0% | 3,0% | 3,0% | 3,0% |
| Polysorbat 20 | 3,0% | 3,0% | 3,0% | 3,0% | 3,0% | 3,0% | 3,0% | 3,0% | 3,0% | 3,0% | 3,0% | 3,0% | 3,0% |
| Sorbitanlaurat | 3,0% | 3,0% | 3,0% | 3,0% | 3,0% | 3,0% | 3,0% | 3,0% | 3,0% | 3,0% | 3,0% | 3,0% | 3,0% |
| Cetearylalkohol | 1,0% | 1,0% | 1,0% | 1,0% | 1,0% | 1,0% | 1,0% | 1,0% | 1,0% | 1,0% | 1,0% | 1,0% | 1,0% |
| Dicaprylylmaleat | 3,0% | 3,0% | 3,0% | 3,0% | 3,0% | 3,0% | 3,0% | 3,0% | 3,0% | 3,0% | 3,0% | 3,0% | 3,0% |
| Dimethicon | 0,5% | 0,5% | 0,5% | 0,5% | 0,5% | 0,5% | 0,5% | 0,5% | 0,5% | 0,5% | 0,5% | 0,5% | 0,5% |
| (C_{12-C15}-Alkyl)-benzoat | 3,0% | 3,0% | 3,0% | 3,0% | 3,0% | 3,0% | 3,0% | 3,0% | 3,0% | 3,0% | 3,0% | 3,0% | 3,0% |
| Methylparaben | 0,2% | 0,2% | 0,2% | 0,2% | 0,2% | 0,2% | 0,2% | 0,2% | 0,2% | 0,2% | 0,2% | 0,2% | 0,2% |
| Propylparaben | 0,2% | 0,2% | 0,2% | 0,2% | 0,2% | 0,2% | 0,2% | 0,2% | 0,2% | 0,2% | 0,2% | 0,2% | 0,2% |
| AVB | 2,5% | 5,0% % | 5,0% % | - | - | - | 2,5% % | 5,0% | 5,0% | 2,5% | 5,0% | 2,5% | 5,0% |
| OMC | - | - | - | 5,0% | 10,0% | 10,0% | 5,0% | 10,0% | 10,0% | - | - | 5,0% | 10,0% |
| EHPT | 5,0% | 5,0% | 10,0% | 5,0% | 5,0% | 10,0% | 5,0% | 5,0% | 10,0% | - | - | - | - |

**Tabelle 2**

| Photostabilität der O/W-Formulierungen | | | | | | | | |
|---|---|---|---|---|---|---|---|---|
| Formulierung | Sonnenschutzfaktor | | | UVA/UVB-Verhältnis | | | HPLC-Analyse | |
| | Anfangswert | % des Anfangswertes nach 30 Min. | | Anfangswertes | % des Anfangswert nach 30 Min. | | % Abbau von AVB | % Abbau von OMC |
| | | Kontrolle | bestrahlt | | Kontrolle | bestrahlt | | |
| 1 A | 17,3±5,2 | 134±14 | 127±8 | 1,00±0,01 | 100,7±0,6 | 98,0±1,0 | 36,9±9,1 | - |
| 1 B | 17,5±2,1 | 126±12 | 155±33 | 1,03±0,02 | 100,6±0,6 | 98,4±0,6 | 13,7±10,1 | - |
| 1 C | 24,0±6,2 | 163±25 | 133±24 | 1,03±0,02 | 100,6±0,6 | 98,4±0,6 | 8,5±11,5 | - |
| 1 D | 23,5±7,7 | 128±18 | 105±4 | 0,76±0,02 | 97,8±2,0 | 100,9±0,8 | - | 29,8±7,8 |
| 1 E | 39,4±19,6 | 125±19 | 140±13 | 0,73±0,03 | 95,9±1,3 | 97,8±0,7 | - | 27,4±14,2 |
| 1 F | 37,7±8,5 | 203±32 | 204±22 | 0,88±0,02 | 96,2±1,6 | 96,7±1,0 | - | 22,0±8,1 |
| 1 G | 22,8±2,9 | 111±9 | 93±11 | 0,90±0,01 | 99,3±0,6 | 97,9±1,1 | 13,1 | 39,3±12,7 |
| 1 H | 82,2±27,4 | 281±22 | 194±30 | 0,95±0,01 | 96,2±1,6 | 94,5±0,6 | 36,9±9,1 | 42,2±7,3 |
| 1 I | 48,2±27,4 | 187±27 | 212±43 | 0,97±0,01 | 99,0±0,1 | 98,0±1,0 | 23,4±13,8 | 30,9±11,1 |
| 1 J | 3,3±0,7 | 107±2 | 56±5 | 1,65±0,13 | 99,4±1,0 | 36,8±6,2 | 69,0±3,0 | - |
| 1 K | 2,8±0,2 | 108±2 | 77±1 | 1,76±0,07 | 96,4±1,6 | 37,6±4,0 | 64,4±11,5 | - |
| 1 L | 17,6±4,7 | 107±5 | 32±4 | 0,82±0,02 | 99,6±1,8 | 70,0±11,6 | 66,4±6,8 | 72,0±6,3 |
| 1 M | 53,0±13,3 | 143±68 | 26±6 | 0,87±0,04 | 98,1±1,7 | 74,3±3,9 | 66,1±1,1 | 70,7±1,9 |

Die Ergebnisse zu den Formulierungen 1 J und 1 K bestätigen die ungenügende Photostabilität von AVB: AVB wurde unter den Versuchsbedingungen überwiegend abgebaut und dementsprechend ergaben sich deutliche Abnahmen des Sonnenschutzfaktors und des UVA/UVB-Verhältnisses. Demgegenüber zeigten die Formulierungen 1 A bis 1 C, die zusätzlich EHPT enthalten, nach dem Belichten keine wesentlichen Änderungen bezüglich des Sonnenschutzfaktors und des UVA/UVB-Verhältnisses, was die stabilisierende Wirkung von EHPT belegt. Die HPLC-Ergebnisse zeigen zwar ebenfalls einen gewissen Abbau von AVB, der aber deutlich geringer war als im Falle der alleinigen Verwendung von AVB und der offenbar die Schutzwirkung nicht beeinträchtigte. Zudem verringerte sich der Anteil an abgebautem AVB bei Erhöhung der Konzentration von AVB und/oder EHPT.

Die Ergebnisse zu den Formulierungen 1 L und 1 M bestätigen, dass OMC zwar ein potenter UVB-Filter, aber in Gegenwart von Dibenzoylmethanen ebenfalls nicht stabil ist. Demgegenüber ist OMC in Gegenwart von EHPT im wesentlichen stabil, wie die Ergebnisse zu den Formulierungen 1 D bis 1 F zeigen. Insbesondere aber wird die Kombination von AVB und OMC durch die Anwesenheit von EHPT deutlich stabilisiert, wie aus den Ergebnissen zu den Formulierungen 1 G bis 1 I ersichtlich ist. Obwohl in der HPLC-Analyse ebenfalls ein gewisser, aber deutlich verringerter Abbau von AVB und OMC gefunden wurde, war der Einfluss auf den Sonnenschutzfaktor und das UVA/UVB-Verhältnis praktisch vernachlässigbar.

### Beispiel 2

### (O/W-Emulsionen mit Phospholipid)

Zur Herstellung der in Tabelle 3 zusammengefassten O/W-Emulsionen mit Phospholipid wurde jeweils Carbomer in Wasser dispergiert, die Dispersion mit Sorbitol 70% und Methylparaben versetzt und die erhaltene wässrige Phase auf 80°C erwärmt. Triceteareth-4-phosphat, Stearinsäure, Cetylalkohol, Dicaprylylmaleat, Dimethicon, (C₁₂₋₁₅-Alkyl)benzoat, Propylparaben, die UV-Filter AVB, OMC und/oder EHPT und eine Lösung von Lecithin in 96%-igem Ethanol wurden in einem separaten Kessel gemischt, auf 80°C erwärmt und dann zur erwärmten wässrigen Phase zugegeben. Das Gemisch wurde während etwa 2 Minuten homogenisiert, dann auf Umgebungstemperatur gekühlt, durch Zugabe von Tromethamin 20% auf einen pH-Wert von 6,0-6,5 eingestellt und nochmals etwa 2 Minuten homogenisiert.

Nach den oben beschriebenen in vitro-Methoden wurde der Einfluss von UV-Licht (30-minütige Bestrahlung mit 30 MED) auf die Schutzeigenschaften und die Stabilität der O/W-Emulsionen untersucht. Die gemessenen Sonnenschutzfaktoren und UVA/UVB-Verhältnisse sowie deren prozentuale Änderungen (bezogen auf den Anfangswert) und der mittels HPLC bestimmte prozentuale Abbau von AVB und/oder OMC sind in Tabelle 4 angegeben. Die angegebenen Werte sind jeweils Mittelwerte (inklusive Streuungen) aus 3 Messungen.

**Tabelle 3**

| O/W-Formulierungen mit Phospholipid: Zusammensetzungen in Gew.-% | | | | | | | | | | | | | |
|---|---|---|---|---|---|---|---|---|---|---|---|---|---|
| Komponenten | 2 A | 2 B | 2 C | 2 D | 2 E | 2 F | 2 G | 2 H | 2 I | 2 J | 2 K | 2 L | 2 M |
| Wasser | 71,1% | 68,6% | 63,6% | 68,6% | 63,6% | 58,6% | 66,1% | 58,6% | 53,5% | 76,1% | 73,6% | 71,1% | 63,6% |
| Carbomer | 0,2% | 0,2% | 0,2% | 0,2% | 0,2% | 0,2% | 0,2% | 0,2% | 0,2% | 0,2% | 0,2% | 0,2% | 0,2% |
| Tromethamin 20% | 0,8% | 0,8% | 0,8% | 0,8% | 0,8% | 0,8% | 0,8% | 0,8% | 0,9% | 0,8% | 0,8% | 0,8% | 0,8% |
| Sorbitol 70% | 3,0% | 3,0% | 3,0% | 3,0% | 3,0% | 3,0% | 3,0% | 3,0% | 3,0% | 3,0% | 3,0% | 3,0% | 3,0% |
| Ethanol 96% | 2,5% | 2,5% | 2,5% | 2,5% | 2,5% | 2,5% | 2,5% | 2,5% | 2,5% | 2,5% | 2,5% | 2,5% | 2,5% |
| Triceteareth-4-phosphat | 4,0% | 4,0% | 4,0% | 4,0% | 4,0% | 4,0% | 4,0% | 4,0% | 4,0% | 4,0% | 4,0% | 4,0% | 4,0% |
| Stearinsäure | 3,0% | 3,0% | 3,0% | 3,0% | 3,0% | 3,0% | 3,0% | 3,0% | 3,0% | 3,0% | 3,0% | 3,0% | 3,0% |
| Cetylalkohol | 0,5% | 0,5% | 0,5% | 0,5% | 0,5% | 0,5% | 0,5% | 0,5% | 0,5% | 0,5% | 0,5% | 0,5% | 0,5% |
| Dicaprylylmaleat | 3,0% | 3,0% | 3,0% | 3,0% | 3,0% | 3,0% | 3,0% | 3,0% | 3,0% | 3,0% | 3,0% | 3,0% | 3,0% |
| Lecithin | 0,5% | 0,5% | 0,5% | 0,5% | 0,5% | 0,5% | 0,5% | 0,5% | 0,5% | 0,5% | 0,5% | 0,5% | 0,5% |
| Dimethicon | 0,5% | 0,5% | 0,5% | 0,5% | 0,5% | 0,5% | 0,5% | 0,5% | 0,5% | 0,5% | 0,5% | 0,5% | 0,5% |
| (C_{12-C15}-Alkyl)-benzoat | 3,0% | 3,0% | 3,0% | 3,0% | 3,0% | 3,0% | 3,0% | 3,0% | 3,0% | 3,0% | 3,0% | 3,0% | 3,0% |
| Methylparaben | 0,2% | 0,2% | 0,2% | 0,2% | 0,2% | 0,2% | 0,2% | 0,2% | 0,2% | 0,2% | 0,2% | 0,2% | 0,2% |
| Propylparaben | 0,2% | 0,2% | 0,2% | 0,2% | 0,2% | 0,2% | 0,2% | 0,2% | 0,2% | 0,2% | 0,2% | 0,2% | 0,2% |
| AVB | 2,5% | 5,0% | 5,0% | - | - | - | 2,5% | 5,0% | 5,0% | 2,5% | 5,0% | 2,5% | 5,0% |
| OMC | - | - | - | 5,0% | 10,0% | 10,0% | 5,0% | 10,0% | 10,0% | - | - | 5,0% | 10,0% |
| EHPT | 5,0% | 5,0% | 10,0% | 5,0% | 5,0% | 10,0% | 5,0% | 5,0% | 10,0% | - | - | - | - |

**Tabelle 4**

| Photostabilität der O/W-Formulierungen mit Phospholipid | | | | | | | | |
|---|---|---|---|---|---|---|---|---|
| Formulierung | Sonnenschutzfaktor | | | UVA/UVB-Verhältnis | | | HPLC-Analyse | |
| | Anfangswert | % des Anfangswertes nach 30 Min. | | Anfangswert | % des Anfangswertes nach 30 Min. | | % Abbau von AVB | % Abbau von OMC |
| | | Kontrolle | bestrahlt | | Kontrolle | bestrahlt | | |
| 2 A | 23,2±8,9 | 113±4 | 107±8 | 1,01±0,01 | 101,0±1,0 | 97,0±1,6 | 4,2±12,9 | - |
| 2 B | 27,7±8,3 | 111±8 | 112±12 | 1,04±0,02 | 100,3±0,6 | 95,9±2,3 | 1,61 | - |
| 2 C | 17,6±2,5 | 108±5 | 102±4 | 1,02±0,01 | 100,0±0,0 | 99,3±0,6 | 0,6±11,0 | - |
| 2 D | 20,4±5,4 | 113±8 | 103±3 | 0,77±0,05 | 98,7±1,2 | 103,1±0,8 | - | 17,4±32,6 |
| 2 E | 43,2±28,9 | 127±1 | 107±7 | 0,74±0,02 | 97,7±0,8 | 101,8±0,9 | - | 27,1±0,6 |
| 2 F | 22,8±4,3 | 122±11 | 106±4 | 0,89±0,02 | 98,5±1,3 | 101,8±3,2 | - | * |
| 2 G | 16,5±0,9 | 119±8 | 105±11 | 0,94±0,01 | 99,3±0,6 | 97,5±0,6 | 26,9±10,7 | 23,0±34,8 |
| 2 H | 25,5±6,0 | 143±22 | 129±24 | 0,98±0,02 | 98,6±1,2 | 96,9±0,1 | 17,8±0,7 | 25,2±0,6 |
| 2 I | 25,1±6,9 | 124±15 | 109±8 | 0,99±0,02 | 98,3±1,6 | 99,3±1,6 | 16,7±7,8 | 28,4±4,1 |
| 2 J | 3,7±0,6 | 101±1 | 49±11 | 1,79±0,12 | 100,4±1,4 | 37,8±8,0 | 57,4±11,9 | - |
| 2 K | 3,0±0,3 | 115±2 | 63±9 | 1,73±0,11 | 97,1±0,9 | 23,4±5,4 | 56,3±8,4 | - |
| 2 L | 13,0±2,4 | 103±5 | 18±3 | 0,81±0,01 | 100,0±0,0 | 52,8±5,2 | 58,0±5,9 | 64,0±4,1 |
| 2 M | 45,5±18,7 | 110±9 | 21±9 | 0,88±0,03 | 98,9±1,1 | 65,3±5,8 | 48,7±4,2 | 54,8±3,4 |

| | | | | | | | | |
|---|---|---|---|---|---|---|---|---|
| * nicht bestimmt | | | | | | | | |

Die Ergebnisse zu den Formulierungen 2 J und 2 K bestätigen die ungenügende Photostabilität von AVB: AVB wurde unter den Versuchsbedingungen überwiegend abgebaut und dementsprechend ergaben sich deutliche Abnahmen des Sonnenschutzfaktors und des UVA/UVB-Verhältnisses. Demgegenüber zeigten die Formulierungen 2 A bis 2 C, die zusätzlich EHPT enthalten, nach dem Belichten keine wesentlichen Änderungen bezüglich des Sonnenschutzfaktors und des UVA/UVB-Verhältnisses, was die stabilisierende Wirkung von EHPT belegt. Die HPLC-Ergebnisse zeigen, dass nur ein sehr geringer Abbau von AVB stattfand und dieser auch niedriger war als im Falle der O/W-Emulsionen ohne Phospholipid (vgl. Formulierungen 1 A bis 1 C von Beispiel 1).

Die Ergebnisse zu den Formulierungen 2 L und 2 M bestätigen, dass OMC zwar ein potenter UVB-Filter, aber in Gegenwart von Dibenzoylmethanen ebenfalls nicht stabil ist. Demgegenüber ist OMC in Gegenwart von EHPT im wesentlichen stabil, wie die Ergebnisse zu den Formulierungen 2 D bis 2 F zeigen. Insbesondere aber wird die Kombination von AVB und OMC durch die Anwesenheit von EHPT deutlich stabilisiert, wie aus den Ergebnissen zu den Formulierungen 2 G bis 2 I ersichtlich ist. Obwohl in der HPLC-Analyse ebenfalls ein gewisser, aber deutlich verringerter Abbau von AVB und OMC gefunden wurde, war der Einfluss auf den Sonnenschutzfaktor und das UVA/UVB-Verhältnis praktisch vernachlässigbar. Zudem war der Anteil an abgebautem AVB und OMC wiederum tendenziell geringer als im Falle entsprechender O/W-Emulsionen ohne Phospholipid (vgl. Formulierungen 1 G bis 1 I von Beispiel 1).

### Beispiel 3

### (W/O-Emulsionen)

Zur Herstellung der in Tabelle 5 zusammengefassten W/O-Emulsionen wurden jeweils Wasser, Natriumlactat-Lösung und Milchsäure gemischt, mit Methylparaben versetzt und die erhaltene wässrige Phase auf 80°C erwärmt. PEG-7-hydriertes Ricinusöl, Methoxy-PEG-22/Dodecylglycol-Copolymer, Arlacel 582, Myristyllactat, Triglycerid mittlerer Kettenlänge, Paraffin, Dimethicon, Propylparaben und die UV-Filter AVB, OMC und/oder EHPT wurden in einem separaten Kessel gemischt, auf 80°C erwärmt und dann zur wässrigen Phase zugegeben. Das Gemisch wurde während etwa 2 Minuten homogenisiert, dann auf Umgebungstemperatur gekühlt, mit Benzylalkohol versetzt und nochmals etwa 2 Minuten homogenisiert.

Nach den oben beschriebenen in vitro-Methoden wurde der Einfluss von UV-Licht (30-minütige Bestrahlung mit 30 MED) auf die Schutzeigenschaften und die Stabilität der O/W-Emulsionen untersucht. Die gemessenen Sonnenschutzfaktoren und UVA/UVB-Verhältnisse sowie deren prozentuale Änderungen (bezogen auf den Anfangswert) und der mittels HPLC bestimmte prozentuale Abbau von AVB und/oder OMC sind in Tabelle 6 angegeben. Die angegebenen Werte sind jeweils Mittelwerte (inklusive Streuungen) aus 3 Messungen.

**Tabelle 5**

| W/O-Formulierungen: Zusammensetzungen in Gew.-% | | | | | | | | | | |
|---|---|---|---|---|---|---|---|---|---|---|
| Komponenten | 3 A | 3 B | 3 C | 3 D | 3 E | 3 F | 3 G | 3 H | 3 I | 3 J |
| Wasser | 56,2% | 53,7% | 48,7% | 51,2% | 43,7% | 38,7% | 61,2% | 58,7% | 56,2% | 48,7% |
| Natriumlactat-Lösung | 2,2% | 2,2% | 2,2% | 2,2% | 2,2% | 2,2% | 2,2% | 2,2% | 2,2% | 2,2% |
| Milchsäure | 0,2% | 0,2% | 0,2% | 0,2% | 0,2% | 0,2% | 0,2% | 0,2% | 0,2% | 0,2% |
| PEG-7-hydr. Ricinusöl | 3,0% | 3,0% | 3,0% | 3,0% | 3,0% | 3,0% | 3,0% | 3,0% | 3,0% | 3,0% |
| Methoxy-PEG-22/Dodecylglycol-Copolymer | 2,0% | 2,0% | 2,0% | 2,0% | 2,0% | 2,0% | 2,0% | 2,0% | 2,0% | 2,0% |
| Arlacel 582 | 3,0% | 3,0% | 3,0% | 3,0% | 3,0% | 3,0% | 3,0% | 3,0% | 3,0% | 3,0% |
| Myristyllactat | 2,5% | 2,5% | 2,5% | 2,5% | 2,5% | 2,5% | 2,5% | 2,5% | 2,5% | 2,5% |
| Triglycerid mittl. Kettenlänge | 5,0% | 5,0% | 5,0% | 5,0% | 5,0% | 5,0% | 5,0% | 5,0% | 5,0% | 5,0% |
| Paraffin | 15,0% | 15,0% | 15,0% | 15,0% | 15,0% | 15,0% | 15,0% | 15,0% | 15,0% | 15,0% |
| Dimethicon | 2,0% | 2,0% | 2,0% | 2,0% | 2,0% | 2,0% | 2,0% | 2,0% | 2,0% | 2,0% |
| Benzylalkohol | 1,0% | 1,0% | 1,0% | 1,0% | 1,0% | 1,0% | 1,0% | 1,0% | 1,0% | 1,0% |
| Methylparaben | 0,2% | 0,2% | 0,2% | 0,2% | 0,2% | 0,2% | 0,2% | 0,2% | 0,2% | 0,2% |
| Propylparaben | 0,2% | 0,2% | 0,2% | 0,2% | 0,2% | 0,2% | 0,2% | 0,2% | 0,2% | 0,2% |
| AVB | 2,5% | 5,0% | 5,0% | 2,5% | 5,0% | 5,0% | 2,5% | 5,0% | 2,5% | 5,0% |
| OMC | - | - | - | 5,0% | 10,0% | 10,0% | - | - | 5,0% | 10,0% |
| EHPT | 5,0% | 5,0% | 10,0% | 5,0% | 5,0% | 10,0% | - | - | - | - |

**Tabelle 6**

| Photostabilität der W/O-Formulierungen | | | | | | | | |
|---|---|---|---|---|---|---|---|---|
| Formu-lierung | Sonnenschutzfaktor | | | UVA/UVB-Verhältnis | | | HPLC-Analyse | |
| | Anfangswert | % des Anfangswertes nach 30 Min. | | Anfangswert | % des Anfangswertes nach 30 Min. | | % Abbau von AVB | % Abbau von OMC |
| | | Kontrolle | bestrahlt | | Kontrolle | bestrahlt | | |
| 3 A | 34,8±13,2 | 141±10 | 142±23 | 1,01±0,02 | 100,0±0,0 | 97,3±0,6 | 17,5±5,8 | - |
| 3 B | 34,1±8,9 | 151±31 | 151±31 | 1,01±0,02 | 100,7±0,6 | 99,7±0,6 | 14,7±12,0 | - |
| 3 C | 62,4±33,7 | 146±11 | 151±12 | 0,99±0,01 | 99,7±0,6 | 99,0±0,1 | 10,8±22,7 | - |
| 3 D | 76,0±34,6 | 130±61 | 154±12 | 0,93±0,01 | 97,1±0,6 | 96,1±0,6 | 19,2±11,8 | 29,9±10,8 |
| 3 E | 45,2±20,9 | 209±114 | 257±108 | 0,97±0,01 | 96,9±1,0 | 94,8±1,1 | 20,3±7,6 | 30,6±5,8 |
| 3 F | 117,4±21,2 | 190±21 | 163±60 | 0,96±0,03 | 97,6±0,6 | 95,7±1,2 | 7,6±2,0 | 16,9±2,1 |
| 3 G | 5,4±1,1 | 105±3 | 29±3 | 1,60±0,19 | 104,6±3,0 | 52,2±7,8 | 65,0±18,9 | - |
| 3 H | 4,7±0,7 | 106±6 | 41±1 | 1,71±0,11 | 100,7±2,5 | 44,2±0,7 | 69,3±8,1 | - |
| 3 I | 40,0±10,0 | 136±48 | 35±6 | 0,85±0,02 | 96,9±0,6 | 68,3±3,2 | 64,6±2,7 | 67,4±1,9 |
| 3 J | 63,8±40,8 | 244±54 | 69±25 | 0,95±0,02 | 93,7±0,9 | 77,3±5,7 | 58,6±5,7 | 65,1±5,5 |

Die Ergebnisse zu den Formulierungen 3 G und 3 H bestätigen die ungenügende Photostabilität von AVB: AVB wurde unter den Versuchsbedingungen überwiegend abgebaut und dementsprechend ergaben sich deutliche Abnahmen des Sonnenschutzfaktors und des UVA/UVB-Verhältnisses. Demgegenüber zeigten die Formulierungen 3 A bis 3 C, die zusätzlich EHPT enthalten, nach dem Belichten keine wesentlichen Änderungen bezüglich des Sonnenschutzfaktors und des UVA/UVB-Verhältnisses, was die stabilisierende Wirkung von EHPT belegt. Die HPLC-Ergebnisse zeigen zwar einen gewissen Abbau von AVB, der aber deutlich geringer war als im Falle der alleinigen Verwendung von AVB und der offenbar die Schutzwirkung nicht beeinträchtigte. Zudem scheint sich der Anteil an abgebautem AVB bei Erhöhung der Konzentration von AVB und/oder EHPT zu verringern.

Die Ergebnisse zu den Formulierungen 3 I und 3 J bestätigen, dass OMC zwar ein potenter UVB-Filter, aber in Gegenwart von Dibenzoylmethanen ebenfalls nicht stabil ist. Durch die Anwesenheit von EHPT wird die Kombination von AVB und OMC jedoch deutlich stabilisiert, wie aus den Ergebnissen zu den Formulierungen 3 D bis 3 F ersichtlich ist. Obwohl in der HPLC-Analyse ebenfalls ein gewisser, aber deutlich verringerter Abbau von AVB und OMC gefunden wurde, war der Einfluss auf den Sonnenschutzfaktor und das UVA/UVB-Verhältnis praktisch vernachlässigbar. Zudem zeigte sich wiederum eine tendenzielle Abnahme des Anteils an abgebautem AVB und OMC bei Erhöhung der EHPT-Konzentration.

## Patentansprüche

1. Verwendung von 2,4-Bis-[(4-(2-ethylhexyloxy)-2-hydroxy]phenyl]-6-(4-methoxyphenyl)-1,3,5-triazin zur Verbesserung der Photostabilität von Dibenzoylmethan-Derivaten.

2. Verwendung nach Anspruch 1, worin das Dibenzoylmethan-Derivat 4-tert-Butyl-4'-methoxydibenzoylmethan ist.

3. Verwendung nach Anspruch 1 oder 2, worin die kosmetische Zusammensetzung ein Verhältnis der UVA-Extinktion zur UVB-Extinktion von 0,6 bis 1,5, vorzugsweise 0,8 bis 1,1, aufweist.

4. Verwendung nach einem der Ansprüche 1 bis 3, worin 2,4-Bis-[[4-(2-ethylhexyloxy)-2-hydroxy]phenyl]-6-(4-methoxyphenyl)-1,3,5-triazin in einer Menge von 0,1 bis 15 Gew.-%, vorzugsweise 0,5 bis 10 Gew.-%, bezogen auf die kosmetische Formulierung, eingesetzt wird.

5. Verwendung nach einem der Ansprüche 1 bis 4, worin die kosmetische Formulierung 0,1 bis 10 Gew.-%, vorzugsweise 0,5 bis 5 Gew.-%, Dibenzoylmethan-Derivat enthält.

6. Verwendung nach einem der Ansprüche 1 bis 5, worin die kosmetische Zusammensetzung eine Emulsion, vorzugsweise eine Öl-in-Wasser-Emulsion oder Wasser-in-Öl-Emulsion ist.

7. Kosmetische Zusammensetzung zur topischen Anwendung zum Schutz gegen UV-Strahlung, insbesondere Sonnenlicht, umfassend einen kosmetisch annehmbaren Träger, mindestens ein Dibenzoylmethan-Derivat, mindestens einen Zimtsäureester und eine wirksame Menge an 2,4-Bis-[[4-(2-ethylhexyloxy)-2-hydroxy]phenyl]-6-(4-methoxyphenyl)-1,3,5-triazin.

8. Verfahren zur Herstellung einer kosmetischen Zusammensetzung zur topischen Anwendung zum Schutz gegen UV-Strahlung, umfassend einen kosmetisch annehmbaren Träger, mindestens ein Dibenzoylmethan-Derivat, mindestens einen Zimtsäureester und eine wirksame Menge an 2,4-Bis-[[4-(2-ethylhexyloxy)-2-hydroxy]phenyl]-6-(4-methoxyphenyl)-1,3,5-triazin, **dadurch gekennzeichnet, dass** man das Dibenzoylmethan-Derivat, den Zimtsäureester und 2,4-Bis-[[4-(2-ethylhexyloxy)-2-hydroxy]phenyl]-6-(4-methoxyphenyl)-1,3,5-triazin in den kosmetisch annehmbaren Träger einbringt.

## Claims

1. Use of 2,4-bis[[4-(2-ethylhexyloxy)-2-hydroxy]phenyl]-6-(4-methoxyphenyl)-1,3,5-triazine for improving the photostability of dibenzoylmethane derivatives.

2. Use according to claim 1, wherein the dibenzoylmethane derivative is 4-tert-butyl-4'-methoxydibenzoylmethane.

3. Use according to claim 1 or 2, wherein the cosmetic composition has a ratio of UVA absorbance to UVB absorbance of from 0.6 to 1.5, preferably from 0.8 to 1.1.

4. Use according to any one of claims 1 to 3, wherein 2,4-bis[[4-(2-ethylhexyloxy)-2-hydroxy]phenyl]-6-(4-methoxyphenyl)-1,3,5-triazine is used in an amount of from 0.1 to 15 % by weight, preferably from 0.5 to 10 % by weight, based on the cosmetic formulation.

5. Use according to any one of claims 1 to 4, wherein the cosmetic formulation contains from 0.1 to 10 % by weight, preferably from 0.5 to 5 % by weight, dibenzoylmethane derivative.

6. Use according to any one of claims 1 to 5, wherein the cosmetic composition is an emulsion, preferably an oil-in-water emulsion or water-in-oil emulsion.

7. Cosmetic composition for topical use for protection against UV radiation, especially sunlight, comprising a cosmetically acceptable carrier, at least one dibenzoylmethane derivative, at least one cinnamic acid ester and an effective amount of 2,4-bis[[4-(2-ethylhexyloxy)-2-hydroxy]phenyl]-6-(4-methoxyphenyl)-1,3,5-triazine.

8. Method for the production of a cosmetic composition for topical use for protection against UV radiation, comprising a cosmetically acceptable carrier, at least one dibenzoylmethane derivative, at least one cinnamic acid ester and an effective amount of 2,4-bis[[4-(2-ethylhexyloxy)-2-hydroxy]phenyl]-6-(4-methoxyphenyl)-1,3,5-triazine, in which method the dibenzoylmethane derivative, the cinnamic acid ester and 2,4-bis[[4-(2-ethylhexyloxy)-2-hydroxy]phenyl]-6-(4-methoxyphenyl)-1,3,5-triazine are incorporated into the cosmetically acceptable carrier.

## Revendications

1. Utilisation de la 2,4-bis-[(4-(2-éthyl-hexyloxy)-2-hydroxy]phényl]-6-(4-méthoxyphényl)-1,3,5-triazine pour l'amélioration de la photostabilité des dérivés du dibenzoylméthane.

2. Utilisation selon la revendication 1, où le dérivé du dibenzoylméthane est le 4-tert-butyl-4'-méthoxydibenzoylméthane.

3. Utilisation selon la revendication 1 ou 2, où la composition cosmétique présente un rapport de l'extinction des UVA à l'extinction des UVB de 0,6 à 1,5, de préférence de 0,8 à 1,1.

4. Utilisation selon la revendication 1 à 3, où la 2,4-bis[[4-(2-éthylhexyloxy)-2-hydroxy)-phényl]-6-(4-méthoxy-phényl)-1,3,5-triazine est utilisée en une quantité de 0,1 à 15 % en masse, de préférence de 0,5 à 10 % en masse, par rapport à la formulation cosmétique.

5. Utilisation selon l'une des revendications 1 à 4, où la formulation cosmétique contient de 0,1 à 10 % en masse, de préférence de 0,5 à 5 % en masse, de dérivé du dibenzoylméthane.

6. Utilisation selon l'une des revendications 1 à 5, où la composition cosmétique est une émulsion, de préférence une émulsion huile dans l'eau ou eau dans l'huile.

7. Composition cosmétique pour l'application topique pour la protection contre le rayonnement UV, en particulier les rayons du soleil, comprenant un véhicule cosmétiquement acceptable, au moins un dérivé du dibenzoylméthane, au moins un ester de l'acide cinnamique et une quantité efficace de 2,4-bis-[(4-(2-éthylhexyloxy)-2-hydroxy]phényl]-6-(4-méthoxyphényl)-1,3,5-triazine.

8. Procédé pour la préparation d'une composition cosmétique pour l'application topique pour la protection contre les rayons UV comprenant un véhicule cosmétiquement acceptable, au moins un dérivé du dibenzoylméthane, au moins un ester de l'acide cinnamique et une quantité efficace de 2,4-bis-[[4-(2-éthyl-hexyloxy)-2-hydroxy]phényll-6-(4-méthoxyphényl)-1,3,5-triazine, **caractérisé en ce qu'**on introduit le dérivé du dibenzoylméthane, l'ester de l'acide cinnamique et la 2,4-bis-[[4-(2-éthylhexyl-oxy)-2-hydroxy]phényl]-6-(4-méthoxyphényl)-1,3,5-triazine dans le véhicule cosmétiquement acceptable.
